# EUROPEAN PATENT SPECIFICATION

(11) **EP 4 640 149 B1**
(45) Date of publication and mention of the grant of the patent: **26.08.2026**
(21) Application number: 25168754.7
(22) Date of filing: 07.04.2025
(51) Int. Cl.: A61B 5/107

(54) **MEDICAL CARE APPARATUS FOR SCANNING**
MEDIZINISCHE PFLEGEVORRICHTUNG ZUM SCANNEN
APPAREIL DE SOINS MÉDICAUX POUR SCANNAGE

(30) Priority: 26.04.2024 JP 2024072284
(43) Date of publication of application: 29.10.2025
(73) Proprietor: J. MORITA MANUFACTURING CORPORATION, Fushimi-ku, Kyoto-shi, Kyoto 6128533 (JP)
(72) Inventor: TANAKA, Tsuyoshi, Kyoto-shi, 612-8533 (JP)
(74) Representative: Müller Hoffmann & Partner

(56) References cited:
- EP-A1- 3 616 599
- JP-A- 2020 034 839
- US-A1- 2021 389 549

## Description

### CROSS REFERENCE TO RELATED APPLICATIONS

This application is based on Japanese Patent Application No. 2024-072284 filed on April 26, 2024 with the Japan Patent Office.

### BACKGROUND

### Field

The present disclosure relates to a medical care apparatus.

### Description of the Background Art

A medical care apparatus which, for example, scan an oral cavity or the like to obtain a three-dimensional shape is known. The device is required to move the focus lens in order to scan the oral cavity or the like. However, moving the focus lens having a mass causes vibrations in the device. Thus, a technique is known which translates a counterweight in a direction opposite the movement of a focus element to substantially balance the movement of the focus lens to reduce the vibrations in the device (e.g., Japanese Patent Laying-Open No. 2015-83978 published JP2015 083978 A)
Further technological background can be found in EP 3 616 599 A1, JP 2020 034839 A and US 2021/389549 A1.

### SUMMARY

Japanese Patent Laying-Open No. 2015-83978 discloses a scanner that mechanically connects, via a translational stage, a focus lens and a counterweight having the same mass and causes the focus lens and the counterweight to move in a linear motion on the translational stage by a mechanical configuration including a motor. However, not only the focus lens, but also the counterweight having the same mass as the focus lens needs to be driven the same distance as the focus lens, which results in an increased power consumption.

The present disclosure is made to solve the above problem, and an object of the present disclosure is to provide a medical care apparatus achieving reduced power consumption.
This is achieved by the claimed subject-matter.

A medical care apparatus according to the present disclosure is a medical care apparatus that scans a three-dimensional shape. The medical care apparatus includes a housing, a lens, a first drive, a counterweight, a second drive, and a controller. The first drive applies, by a first magnetic circuit, a force to the lens in a first linear motion direction to cause the lens to move in a linear motion in the first linear motion direction. The counterweight has a mass N times a mass of the lens, with N>1. The second drive applies, by a second magnetic circuit, a force to the counterweight in a second linear motion direction on the same linear line as the first linear motion direction to cause the counterweight to move in a linear motion in the second linear motion direction. The controller controls driving of each of the first drive and the second drive. The controller causes the counterweight to move in a motion in a relative direction with respect to the lens at a driving amplitude 1/N times a driving amplitude of the lens.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

Fig. 1 is a schematic diagram showing a configuration of a three-dimensional scanner.
Fig. 2 is a schematic diagram showing a configuration of a handpiece.
Fig. 3 is a diagram for illustrating a positional relationship between a lens and a counterweight in the three-dimensional scanner.
Fig. 4 is a perspective view of a linear motor.
Fig. 5 is a diagram for illustrating driving of the lens and the counterweight.
Fig. 6 is a diagram for illustrating design parameters for the three-dimensional scanner.

### DETAILED DESCRIPTION

A medical care apparatus is now described with reference to the accompanying drawings. A three-dimensional scanner used for a dental practice is described as one example of the medical care apparatus. The three-dimensional scanner is an intraoral scanner for obtaining a three-dimensional shape of teeth in an oral cavity. The three-dimensional scanner is not limited to intraoral scanners and applicable to other three-dimensional scanners having a similar configuration, for example, a scanner for obtaining a three-dimensional shape of an inside of an outer ear by capturing images of the inside of the outer ear, besides the oral cavity. Note that the fields of use of the three-dimensional scanner are not limited to dentistry, and the three-dimensional scanner can be used in all possible medical fields such as ophthalmology, otorhinolaryngology, radiology, and veterinary medicine. The terms in medical practices in the present disclosure encompass the meanings of medical practices and treatments too.

### [Configuration of Three-Dimensional Scanner]

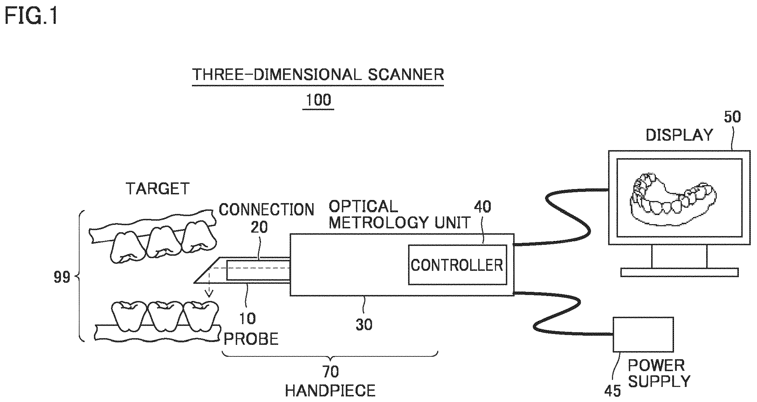

Fig. 1 is a schematic diagram showing a configuration of a three-dimensional scanner 100. As shown in Fig. 1, three-dimensional scanner 100 includes a handpiece 70, a controller 40, a display 50, and a power supply 45. Handpiece 70 is a handheld member and includes a probe 10, a connection 20, and an optical metrology unit 30. Controller 40 is included inside the optical metrology unit 30.

Probe 10 is put into an oral cavity and projects light having a pattern (hereinafter, also, simply referred to as a pattern) onto a target 99 such as teeth. Probe 10 guides the reflected light from target 99, having the pattern projected thereon, to optical metrology unit 30. Probe 10 is detachable from optical metrology unit 30. Due to this, as infection control measures, an operator can detach only probe 10 that may contact biological substances, from optical metrology unit 30, for sterilization (e.g., a process under high-temperature and high-humidity environment).

Connection 20 is a part of optical metrology unit 30, projecting from optical metrology unit 30 and having a shape that can engage with the root of probe 10. Connection 20 includes a lens system for guiding the light captured by probe 10 to optical metrology unit 30, and optical components such as a glass coverslip, an optical filter, and a wave plate (a quarter-wave plate).

Optical metrology unit 30 projects a pattern onto target 99 via probe 10 and captures images of the projected pattern. Note that the optical metrology unit 30 uses the principle of a focusing method to obtain a three-dimensional shape, as described below. However, optical metrology unit 30 may use the principle of a confocal method or the like to obtain a three-dimensional shape. In other words, optical metrology unit 30 may use any principle insofar as optical metrology unit 30 can change the projection pattern or the focus of the optical sensor and use an optical approach to obtain a three-dimensional shape.

Controller 40 controls the operation of optical metrology unit 30 and processes the images captured by optical metrology unit 30 to obtain a three-dimensional shape. Although not shown, controller 40 includes a central processing unit (CPU) as a control center, a read only memory (ROM) storing programs and control data for the CPU to operate with, a random access memory (RAM) that functions as a work area for the CPU, and an input/output interface for providing consistency of signals with peripheral devices. Controller 40 can also output the obtained three-dimensional shape to display 50 and allow input of information such as the settings of optical metrology unit 30 by an input device.

Note that at least a part of calculation for processing the captured images to obtain the three-dimensional shape may be implemented as software by the CPU of controller 40 or implemented as hardware that performs processes separate from the CPU. At least some of the processes by the CPU or the hardware may be performed by a PC or the like, which is external to optical metrology unit 30. While Fig. 1 depicts the components (optical metrology unit 30, power supply 45, display 50) of three-dimensional scanner 100 as being wired by cables (the thick lines in the figure), some or all of the wires may be connected by wireless communications.

Display 50 shows a result of measurement of the three-dimensional shape of target 99 obtained by controller 40. Display 50 can also show other information such as the settings information of optical metrology unit 30, patient information, the activation-related state of the scanner, an instruction manual, and a help screen. For example, a stationary liquid crystal display, and a head-mounted or glasses-type wearable display are applicable to display 50. Moreover, there may be a number of displays 50 and a result of measurement of the three-dimensional shape and other information may be simultaneously displayed on or extended across the displays 50.

Power supply 45 supplies power to optical metrology unit 30 and controller 40. While power supply 45 may be provided external to handpiece 70 as shown in Fig. 1, power supply 45 may be provided inside the handpiece 70. Moreover, a number of power supplies 45 may be provided so that they can individually supply power to controller 40, optical metrology unit 30, and display 50.

### [Configuration of Handpiece]

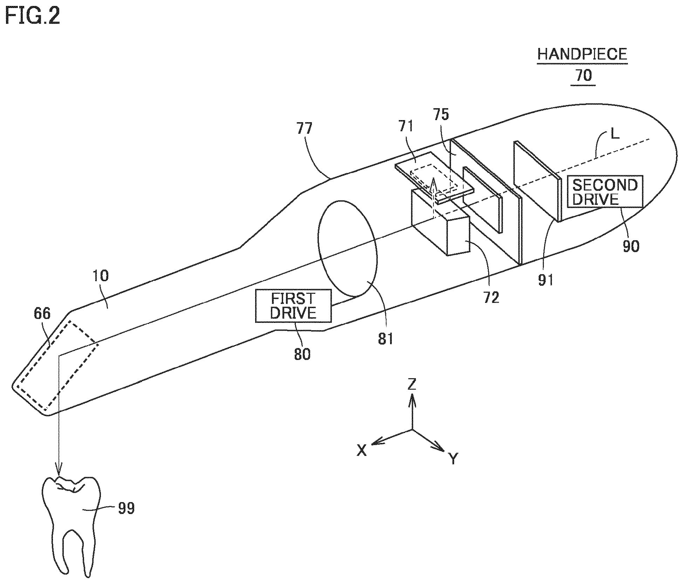

Fig. 2 is a schematic diagram showing a configuration of a handpiece 70. Note that the members inside the handpiece 70 shown in Fig. 2 are accommodated in optical metrology unit 30 shown in Fig. 1. As shown in Fig. 2, handpiece 70 includes a light source 71, a lens 81, an optical sensor 75, and a controller 40 inside the housing 77. Besides these components, handpiece 70 may include a beam splitter for decoupling the light from light source 71 toward target 99 and the light from target 99 toward optical sensor 75, and a reflector for reflecting light onto target 99. Note that, in the description below, for convenience of illustration, L indicates a phantom linear line representing a direction of a linear motion of lens 81, and the axis parallel to linear line L will be referred as X axis, the axis that is perpendicular to linear line L and upward relative to the plane of the drawing of Fig. 2 will be referred to as Z axis, and the axis perpendicular to X and Z axes will be referred to as Y axis.

Light output from light source 71 passes through lens 81, is emitted to target 99, and reflected by target 99. The light reflected by target 99 passes through lens 81, again, and is detected by optical sensor 75. To obtain a three-dimensional shape using the technique of the focusing method, light passing through a pattern-generating element (not shown) disposed between lens 81 and target 99 is projected onto target 99. As lens 81 moves in a linear motion on a straight line (e.g., linear line L in the figure), the focal position of the projection pattern changes. For each change, optical sensor 75 detects light from target 99. Controller 40 described above calculates geometric information of target 99, based on the position of lens 81 and a result of detection by optical sensor 75 when lens 81 is at that position.

As lens 81 moves in a linear motion in the direction of linear line L (X-axis direction), the center of gravity of handpiece 70 moves by the mass of lens 81, which is transferred, as vibrations, to a hand of the user holding handpiece 70. In order to counteract the vibrations, handpiece 70 further includes a counterweight 91 inside the housing 77. Counterweight 91 is disposed behind the optical sensor 75 in X-axis direction in a manner not blocking the optical path between target 99 and lens 81 and the optical path between lens 81 and optical sensor 75.

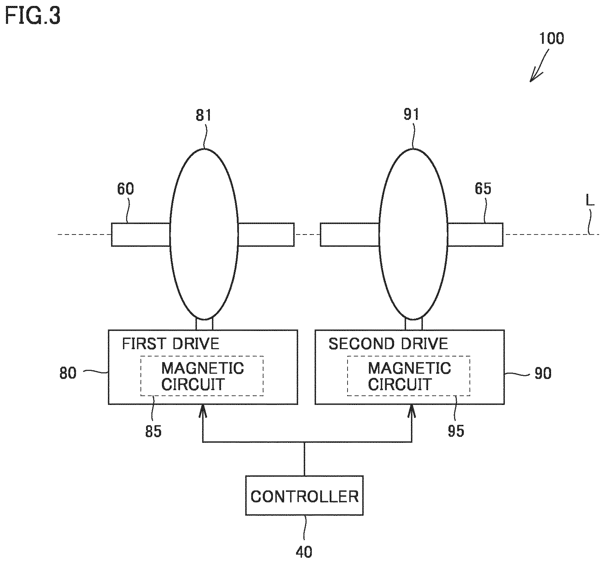

Fig. 3 is a schematic diagram for illustrating a positional relationship between lens 81 and counterweight 91 in three-dimensional scanner 100. As shown in Fig. 3, lens 81 is supported by a linear guide 60 parallel to linear line L so as to move in a linear motion in the direction of linear line L. Although not shown, linear guide 60 is fixed by housing 77. Furthermore, lens 81 is connected to a magnetic circuit 85 of a first drive 80. First drive 80 constitutes a linear motor, by magnetic circuit 85, for causing lens 81 to move in a linear motion in the direction of linear line L.

Counterweight 91 is a weight that has a mass N times, with N>1, the mass of lens 81 and is driven by a second drive 90 at a driving amplitude 1/N times the driving amplitude of lens 81.
Three-dimensional scanner 100 can reduce the power consumed by counterweight 91, as compared to the case of driving a counterweight that has the same mass as the lens by the same distance as the lens. Counterweight 91 is disposed on the same linear line L as lens 81 and supported by a linear guide 65 that is parallel to linear line L. Linear guide 65 may be a separate member from linear guide 60. Furthermore, counterweight 91 is connected to a magnetic circuit 95 of second drive 90. Second drive 90 constitutes a linear motor, by magnetic circuit 95, for causing counterweight 91 to move in a linear motion in the direction of linear line L.

First drive 80 and second drive 90 are each controlled by controller 40. While first drive 80 and second drive 90 are indicated to be controlled by a common controller 40, it should be noted that the first drive 80 and second drive 90 may be controlled by different controllers.

As first drive 80 causes lens 81 to move in a linear motion in the direction of linear line L, second drive 90 causes counterweight 91 to move in a linear motion at a driving amplitude 1/N times the driving amplitude of lens 81, in a relative direction with respect to lens 81, i.e. in a relatively opposite direction as the lens 81. For example, where the mass of counterweight 91 is twice (N = 2) the mass of lens 81, as lens 81 moves 5 mm on linear line L in a direction toward target 99, counterweight 91 moves 2.5 mm on linear line L in a direction away from target 99. As lens 81 moves 5 mm on linear line L in a direction away from target 99, counterweight 91 moves 2.5 mm on linear line L in a direction toward target 99. In other words, the driving amplitude of counterweight 91 is half (1/2) the driving amplitude of lens 81.

As such, causing counterweight 91, which has the mass N times, with N>1, the mass of lens 81, to move in a linear motion in the relative/opposite direction with respect to lens 81 at the driving amplitude 1/N times the driving amplitude of lens 81 results in lens 81 and counterweight 91 having the same momentum. Therefore, the bias in the center of gravity of handpiece 70 caused by lens 81 being driven can be cancelled by counterweight 91, counteracting the vibration of handpiece 70.

### [Configuration of Linear Motor]

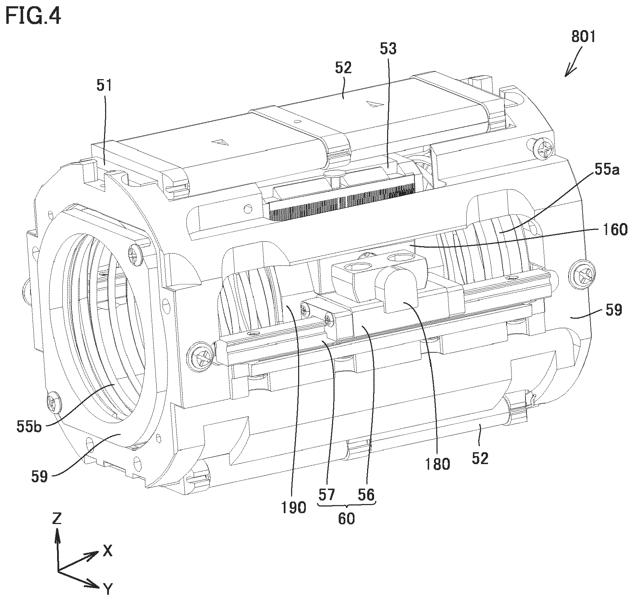

Next, a specific configuration of the linear motor is described with reference to the accompanying drawings. Fig. 4 is a perspective view of a linear motor 801. Note that, in the example of Fig. 4, among other linear motors, only a configuration of linear motor 801 corresponding to first drive 80 will be described. However, a linear motor corresponding to second drive 90 has the same configuration as linear motor 801. In other words, for the linear motor corresponding to second drive 90, lens 81 is replaced with counterweight 91 in the example of Fig. 4, but the other configuration of the linear motor is the same as the configuration of linear motor 801.

Linear motor 801 has a magnetic circuit 85 including a yoke 51, a coil 52, and permanent magnets 53. Linear motor 801 also has a mover 190 for holding lens 81, around the outer periphery of lens 81, and permanent magnets 53 are provided above and below the mover 190 in the figure. Note that the yoke 51 and coil 52 which are disposed opposite the permanent magnet 53 constitute a stator for linear motor 801. Permanent magnets 53 are disposed to secure mover 190 so that, for example, the north pole is oriented in the positive direction of X axis and the south pole is oriented in the negative direction of X axis.

Mover 190 has an opening in the direction of linear motion and holds lens 81 (optical component) in the opening. Thus, the optical path between target 99 and optical sensor 75 passes through the opening. One end of mover 190 holding lens 81 abuts a spring 55a and the other end of mover 190 abuts a spring 55b.

Spring 55a and spring 55b are disposed around the outer periphery of lens 81 in a manner not blocking the optical path at the center of lens 81. Spring 55a and spring 55b correspond to one example of an elastic member. For example, coil springs are applied to spring 55a and spring 55b. Note that the elastic member is not limited to springs, and any member that deforms upon application of a force and returns to the original shape upon the release of the force, such as a rubber, can be applied.

Spring 55a and spring 55b each have one end abutting mover 190 and the other end fixed by a housing 59 of linear motor 801. Furthermore, spring 55a and spring 55b are held within housing 59 so that they are allowed to deform in X direction and are hardly deform in Y-Z direction. Spring 55a and spring 55b disposed as such provide mover 190 with resiliency in the direction of linear motion.

Linear motor 801 has two linear guides 60 in parallel to each other on the outer periphery of mover 190, the linear guides 60 each configured of a rail 57 and a block 56. The two linear guides 60 are disposed at different positions on the outer periphery side of mover 190. Specifically, the two linear guides 60 are disposed in parallel to each other at positions that are rotationally symmetric to each other about the optical axis (linear line L) in parallel to the direction of the linear motion of lens 81 and passing through lens 81, as the axis of rotation. Note that the two linear guides 60 are not necessarily be provided, and one linear guide 60 or three or more linear guides 60 may be provided.

Block 56 supports mover 190 and lens 81 and is engaged with rail 57. Block 56 moves in the direction of linear line along rail 57, thereby causing lens 81 to move in a reciprocating linear motion. A viscous lubricant such as grease may be applied or a rolling bearing such as a ball or a roller may be disposed, between block 56 and the connection surface of rail 57.

Lens 81 and mover 190 are supported together by linear guide 60 via a support 180 and a holding member 160, in a manner enabling the reciprocating linear motion of lens 81. Specifically, holding member 160 is provided on a portion of mover 190 holding lens 81. Support 180 is screwed to a portion of block 56 that moves on rail 57. Support 180 and holding member 160 are engaged with each other.

### [Driving of Lens and Counterweight]

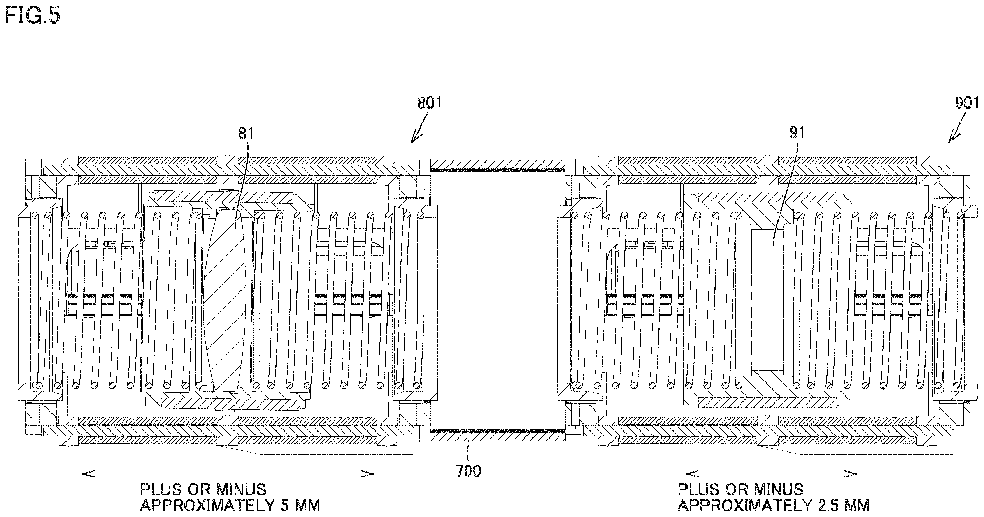

Next, the driving of lens 81 and counterweight 91 is described in detail. Fig. 5 is a diagram for illustrating driving of lens 81 and counterweight 91. As shown in Fig. 5, linear motor 801 for driving lens 81 and a linear motor 901 for driving counterweight 91 are linearly connected via a connecting member 700. Then, counterweight 91 having the mass N times, with N>1, the mass of lens 81 is caused to move in a linear motion at a driving amplitude 1/N times the driving amplitude of lens 81 in the relative/opposite direction with respect to lens 81, and the bias in the center of gravity of handpiece 70, caused by the driving of lens 81, is thereby cancelled, counteracting the vibrations of handpiece 70.

Specifically, suppose that, where the mass of lens 81 is approximately 10 g and the driving amplitude of lens 81 is plus or minus approximately 5 mm, the mass of counterweight 91 is approximately 20 g, which is approximately twice (N = 2) the mass of lens 81, and the driving amplitude of counterweight 91 is plus or minus approximately 2.5 mm, which is approximately half the driving amplitude of lens 81. Driving lens 81 and counterweight 91 in such a manner results in the momentum of lens 81 and the momentum of counterweight 91 being equal, counteracting the vibrations of handpiece 70. Furthermore, it can be seen, from (Equation 1), which is the momentum equation for the linear motor, that the parameters for the motion system are determined by Mₙ (mass), Dₙ (viscous modulus), and Kₙ (spring constant) only. Parameters for a control input F (xₙ, t) are determined by a drive frequency f_{d} and a driving amplitude A_{d} of the linear motor.
[MATH 1] ${M}_{n} \frac{{d}^{2} {x}_{n}}{{dt}^{2}} + {D}_{n} \frac{{dx}_{n}}{dt} + {K}_{n} \left(x\right) x = F {\left({x}_{n}, t\right)}_{drive}$

Since driving amplitude A_{d} of the linear motor is proportional to the current flowing through the coil and the power consumed by the linear motor is Joule loss caused by the current flowing through the coil, the power consumed by the linear motor is proportional to the square of the current flowing through the coil. Therefore, the power consumed by the linear motor is proportional to the square of driving amplitude A_{d} of the linear motor. In other words, if driving amplitude A_{d} of the linear motor is reduced by a factor of N, the power consumed by the linear motor can be reduced by a factor of N².

As mentioned earlier, if the driving amplitude of counterweight 91 is plus or minus approximately 2.5 mm, which is approximately half the driving amplitude of lens 81, the power consumed by linear motor 901 driving counterweight 91 can be reduced by a factor of (4). In particular, three-dimensional scanner 100 for scanning an oral cavity is computationally intensive at controller 40 provided within handpiece 70, and the heat generated from the CPU of controller 40 may be problematic. Therefore, in three-dimensional scanner, reducing the driving amplitude of counterweight 91 by a factor of N reduces the power consumed by linear motor 901 by a factor of N², thereby suppressing linear motor 901 from generating heat and the heat generation of the entire device is reduced.

Since the power consumed by linear motor 901 is Joule loss caused by the current flowing through coil 52, reducing the size of linear motor 901 can reduce the resistance of coil 52, thereby further reducing the power consumed by linear motor 901. Moreover, if the degree of vibration of handpiece 70 can be determined, from a sense of holding handpiece 70, to be sufficiently small, further reduction of the driving amplitude of counterweight 91 can even further reduce the power consumed by linear motor 901.

### [Design Parameters]

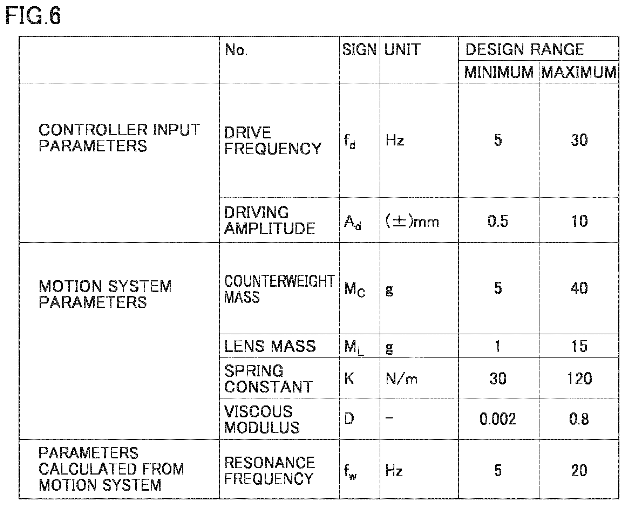

Next, design parameters for three-dimensional scanner 100 are described. Fig. 6 is a diagram for illustrating design parameters of three-dimensional scanner 100. As shown in Fig. 6, the design parameters include control input parameters, motion system parameters, and parameters calculated from the motion system. The control input parameters are parameters for the control input F (xₙ, t) and include drive frequency f_{d} [unit: Hz] and driving amplitude A_{d} [unit: mm] of the linear motor.

As can be seen from (Equation 1) of the momentum equation described above, the motion system parameters include Mₙ (mass) [unit: g], Dₙ (viscous modulus), and Kₙ (spring constant) [unit: N/m]. Note that Mₙ (mass) includes mass M_{C} of counterweight 91 and mass M_{L} of lens 81. The parameters calculated from the motion system include a resonance frequency f_{W} [unit: Hz].

Values of the design parameters shown in Fig. 6 are one example of three-dimensional scanner 100 for scanning an oral cavity. For example, drive frequency f_{d} of linear motors 801 and 901 is 5 Hz to 30 Hz. An increase of drive frequency f_{d} of linear motors 801 and 901 increases the speed of scanning the oral cavity, failing the calculation by controller 40 to catch up with the speed, and the heat generation from controller 40 may be enhanced. An increase of drive frequency f_{d} of linear motors 801 and 901 may also cause problems with the durability of linear motors 801 and 901 themselves. Conversely, a reduction of drive frequency f_{d} of linear motors 801 and 901 slows down the speed of scanning of the oral cavity, which reduces the data speed from three-dimensional scanner 100.

Driving amplitude A_{d} of linear motors 801 and 901 is plus or minus 0.5 mm to plus or minus 10 mm. An increase of the value of the driving amplitude A_{d} of linear motors 801 and 901 extends the depth of field of three-dimensional scanner 100, which is, however, restricted by the lengths of linear motors 801 and 901 in the directions of the linear motions. Driving amplitude A_{d} of linear motor 801 is the driving amplitude of lens 81, and driving amplitude A_{d} of linear motor 901 is the driving amplitude of counterweight 91. In view of the design parameters shown in Fig. 6, the driving amplitude of counterweight 91 is, for example, greater than or equal to (1/20) times the driving amplitude of lens 81 and less than 1 times the driving amplitude of lens 81.

Mass M_{L} of lens 81 is 1 g to 15 g. Mass M_{L} of lens 81 is determined by an optical design. In contrast, mass M_{C} of counterweight 91 is 5 g to 40 g. Mass M_{C} of counterweight 91 needs to be heavier than mass M_{L} of lens 81. Therefore, the use of a material having the same density as lens 81 ends up an increase in size of counterweight 91. Thus, preferably, a material used in counterweight 91 has a greater density than lens 81.

Spring constant Kₙ is 30 N/m to 120 N/m. Viscous modulus Dₙ is 0.002 to 0.8. Resonance frequency f_{W} is 5 Hz to 20 Hz. Resonance frequency f_{W} of linear motor 801 (first drive 80) and resonance frequency f_{W} of linear motor 901 (second drive 90) may be the same. However, since the handheld three-dimensional scanner 100 may be tilted for use, preferably, resonance frequency f_{W} of linear motor 801 is higher than resonance frequency f_{W} of linear motor 901.

### [Variations]

The medical care apparatus according to the present disclosure is not limited to the configuration described above, and other various variations and applications are possible. In the following, variations applicable to the present disclosure are described.

### (Object)

In three-dimensional scanner 100, lens 81 and counterweight 91 move in a linear motion in relative directions, as shown in Fig. 2. However, counterweight 91 may have the function of a lens.

### (Locations of Respective Members of Drive)

In three-dimensional scanner 100, spring 55a and spring 55b are disposed to have lens 81 sandwiched therebetween in the direction of linear line L, as shown in Fig. 4. However, the number of springs and their locations are not limited thereto. For example, multiple springs may be disposed around the outer periphery of lens 81 in a manner not blocking the optical path at the center of lens 81. Furthermore, while spring 55a and spring 55b are disposed to have lens 81 sandwiched therebetween, a spring may be disposed only on one side of lens 81.

### (Drive)

In three-dimensional scanner 100, the drive uses the magnetic circuit including the magnet, the coil, and the yoke to apply a force to lens 81 and counterweight 91 in the direction of linear line L, as shown in Figs. 3 to 5. However, the drive may use a configuration different from such a magnetic circuit to apply the force to lens 81 and counterweight 91 in the direction of linear line L.

In three-dimensional scanner 100, the drive utilizes the resonance phenomenon, due to a response of the motion system, to cause lens 81 and counterweight 91 to move in a linear motion at a constant cycle in the direction of linear line L, as shown in Figs. 3 to 5. However, it is not necessarily required to employ the springs. Without springs, the current may be kept flowing through the magnetic circuit for the progressive motion of an object, and the current flowing through the magnetic circuit may be stopped to stop the object.

The first drive and the second drive may both utilize the resonance phenomenon due to a response of the motion system, none of them may utilize the resonance phenomenon due to a response of the motion system, or either one of the first drive and the second drive may utilize the resonance phenomenon due to a response of the motion system.

### (Applications to Other Uses)

Above, a three-dimensional scanner that can be used for a dental practice has been described as one example of the medical care apparatus. However, the medical care apparatus is applicable to other uses too. For example, as the medical care apparatus, a cutting device may be applied which uses a cutting tool (e.g., a scaler tip, a file for root canal therapy) to cut or trim a target into a desired shape.

The above description should be considered in all aspects as illustrative and not restrictive. The scope of the present invention is defined by the appended claims, and not by the description above. Note that the above configurations can be combined as appropriate.

## Claims

1. A medical care apparatus (100) for scanning a three-dimensional shape, comprising:
a housing (77);
a lens (81);
a first drive (80) configured to apply, by a first magnetic circuit (85), a force to the lens (81) in a first linear motion direction to cause the lens (81) to move in a linear motion in the first linear motion direction;
a counterweight (91);
a second drive (90) configured to apply, by a second magnetic circuit (95), a force to the counterweight (91) in a second linear motion direction on the same linear line (L) as the first linear motion direction to cause the counterweight (91) to move in a linear motion in the second linear motion direction; and
a controller (40) configured to control driving of each of the first drive (80) and the second drive (90),
**characterized in that**
the counterweight (91) has a mass N times a mass of the lens (81), with N>1; and
the controller (40) causes the counterweight (91) to move in a motion in a relative direction with respect to the lens (81) at a driving amplitude 1/N times a driving amplitude of the lens (81).

2. The medical care apparatus (100) according to claim 1, wherein
at least one of the first drive (80) and the second drive (90) includes an elastic member that provides resiliency in a direction of linear motion.

3. The medical care apparatus (100) according to claim 2, wherein
the first drive (80) includes the elastic member in front and behind the lens (81) in the first linear motion direction, and
the second drive (90) includes the elastic member in front and behind the counterweight (91) in the second linear motion direction.

4. The medical care apparatus (100) according to claim 2 or 3, wherein
the elastic member is a coil spring, and
an inner diameter of the coil spring is an optical path of the lens (81).

5. The medical care apparatus (100) according to any one of claims 1 to 4, wherein
the controller (40) is configured to drive the first drive (80) and the second drive (90) at the same drive frequency.

6. The medical care apparatus (100) according to any one of claims 1 to 4, wherein the first drive (80) has a higher resonance frequency than the second drive (90).

7. The medical care apparatus (100) according to any one of claims 1 to 6, wherein
the driving amplitude of the counterweight (91) is greater than or equal to 1/20 times the driving amplitude of the lens (81) and less than 1 times the driving amplitude of the lens (81).

8. The medical care apparatus (100) according to any one of claims 1 to 7, wherein
the first magnetic circuit (85) and the second magnetic circuit (95) include a magnet (53), a coil (52), and a yoke (51).

9. The medical care apparatus (100) according to any one of claims 1 to 8, wherein
the medical care apparatus (100) is of a handheld type.

## Patentansprüche

1. Medizinische Pflegevorrichtung (100) zum Scannen einer dreidimensionalen Form, aufweisend:
ein Gehäuse (77);
eine Linse (81);
einen ersten Antrieb (80), der konfiguriert ist, um durch einen ersten Magnetkreis (85) eine Kraft auf die Linse (81) in einer ersten linearen Bewegungsrichtung auszuüben, um zu bewirken, dass sich die Linse (81) in einer linearen Bewegung in der ersten linearen Bewegungsrichtung bewegt;
ein Gegengewicht (91);
einen zweiten Antrieb (90), der konfiguriert ist, um durch einen zweiten Magnetkreis (95) eine Kraft auf das Gegengewicht (91) in einer zweiten linearen Bewegungsrichtung auf derselben linearen Linie (L) wie die erste lineare Bewegungsrichtung auszuüben, um zu bewirken, dass sich das Gegengewicht (91) in einer linearen Bewegung in der zweiten linearen Bewegungsrichtung bewegt; und
eine Steuerung (40), die konfiguriert ist, um den Antrieb von jedem des ersten Antriebs (80) und des zweiten Antriebs (90) zu steuern,
**dadurch gekennzeichnet, dass**
das Gegengewicht (91) eine Masse aufweist, die das N-fache einer Masse der Linse (81) ist, wobei N>1; und
die Steuerung (40) bewirkt, dass sich das Gegengewicht (91) in einer Bewegung in einer relativen Richtung in Bezug auf die Linse (81) mit einer Antriebsamplitude bewegt, die das 1/Nfache einer Antriebsamplitude der Linse (81) ist.

2. Medizinische Pflegevorrichtung (100) nach Anspruch 1, wobei
mindestens einer von dem ersten Antrieb (80) und dem zweiten Antrieb (90) ein elastisches Element beinhaltet, das eine Nachgiebigkeit in einer Richtung der linearen Bewegung bereitstellt.

3. Medizinische Pflegevorrichtung (100) nach Anspruch 2, wobei
der erste Antrieb (80) das elastische Element vor und hinter der Linse (81) in der ersten linearen Bewegungsrichtung beinhaltet, und
der zweite Antrieb (90) das elastische Element vor und hinter dem Gegengewicht (91) in der zweiten linearen Bewegungsrichtung beinhaltet.

4. Medizinische Pflegevorrichtung (100) nach Anspruch 2 oder 3, wobei
das elastische Element eine Schraubenfeder ist, und
ein Innendurchmesser der Schraubenfeder ein optischer Pfad der Linse (81) ist.

5. Medizinische Pflegevorrichtung (100) nach einem der Ansprüche 1 bis 4, wobei
die Steuerung (40) konfiguriert ist, um den ersten Antrieb (80) und den zweiten Antrieb (90) mit der gleichen Antriebsfrequenz anzutreiben.

6. Medizinische Pflegevorrichtung (100) nach einem der Ansprüche 1 bis 4, wobei der erste Antrieb (80) eine höhere Resonanzfrequenz als der zweite Antrieb (90) hat.

7. Medizinische Pflegevorrichtung (100) nach einem der Ansprüche 1 bis 6, wobei
die Antriebsamplitude des Gegengewichts (91) größer als oder gleich dem 1/20-fachen der Antriebsamplitude der Linse (81) und kleiner als das 1-fache der Antriebsamplitude der Linse (81) ist.

8. Medizinische Pflegevorrichtung (100) nach einem der Ansprüche 1 bis 7, wobei
der erste Magnetkreis (85) und der zweite Magnetkreis (95) einen Magneten (53), eine Spule (52) und ein Joch (51) beinhalten.

9. Medizinische Pflegevorrichtung (100) nach einem der Ansprüche 1 bis 8, wobei
die medizinische Pflegevorrichtung (100) von einem handgehaltenen Typ ist.

## Revendications

1. Appareil de soins médicaux (100) pour scanner une forme tridimensionnelle comprenant :
un logement (77) ;
une lentille (81) ;
un premier entraînement (80) configuré pour appliquer, par un premier circuit magnétique (85), une force à la lentille (81) dans une première direction de mouvement linéaire pour amener la lentille (81) à se déplacer dans un mouvement linéaire dans la première direction de mouvement linéaire ;
un contrepoids (91) ;
un second entraînement (90) configuré pour appliquer, par un second circuit magnétique (95), une force au contrepoids (91) dans une seconde direction de mouvement linéaire sur la même ligne linéaire (L) que la première direction de mouvement linéaire pour amener le contrepoids (91) à se déplacer dans un mouvement linéaire dans la seconde direction de mouvement linéaire ; et
un dispositif de commande (40) configuré pour commander l'entraînement de chacun du premier entraînement (80) et du second entraînement (90),
**caractérisé en ce que**
le contrepoids (91) a une masse N fois une masse de la lentille (81), avec N>1 ; et
le dispositif de commande (40) amène le contrepoids (91) à se déplacer en un mouvement dans une direction relative par rapport à la lentille (81) à une amplitude d'entraînement 1/N fois une amplitude d'entraînement de la lentille (81).

2. Appareil de soins médicaux (100) selon la revendication 1, dans lequel
au moins l'un parmi le premier entraînement (80) et le second entraînement (90) comporte un organe élastique qui procure une élasticité dans une direction de mouvement linéaire.

3. Appareil de soins médicaux (100) selon la revendication 2, dans lequel
le premier entraînement (80) comporte l'organe élastique devant et derrière la lentille (81) dans la première direction de mouvement linéaire, et
le second entraînement (90) comporte l'organe élastique devant et derrière le contrepoids (91) dans la seconde direction de mouvement linéaire.

4. Appareil (100) de soins médicaux selon la revendication 2 ou 3, dans lequel
l'organe élastique est un ressort hélicoïdal, et
un diamètre interne du ressort hélicoïdal est un chemin optique de la lentille (81).

5. Appareil de soins médicaux (100) selon l'une des revendications 1 à 4, dans lequel
le dispositif de commande (40) est configuré pour entraîner le premier entraînement (80) et le second entraînement (90) à la même fréquence d'entraînement.

6. Appareil (100) de soins médicaux selon l'une des revendications 1 à 4, dans lequel le premier entraînement (80) présente une fréquence de résonance plus élevée que le second entraînement (90).

7. Appareil (100) de soins médicaux selon l'une des revendications 1 à 6, dans lequel
l'amplitude d'entraînement du contrepoids (91) est supérieure ou égale à 1/20 fois l'amplitude d'entraînement de la lentille (81) et inférieure à 1 fois l'amplitude d'entraînement de la lentille (81).

8. Appareil (100) de soins médicaux selon l'une des revendications 1 à 7, dans lequel
le premier circuit magnétique (85) et le second circuit magnétique (95) comportent un aimant (53), une bobine (52) et une culasse (51).

9. Appareil (100) de soins médicaux selon l'une des revendications 1 à 8, dans lequel
l'appareil (100) de soins médicaux est de type portatif.
